# EUROPÄISCHE PATENTSCHRIFT

(11) **EP 1 365 714 B1**
(45) Veröffentlichungstag und Bekanntmachung des Hinweises auf die Patenterteilung: **27.06.2007**
(21) Anmeldenummer: 02704669.7
(22) Anmeldetag: 24.01.2002
(51) Int. Cl.: A61F 9/013

(54) **KLINGENHALTERUNG FÜR EIN CHIRURGISCHES INSTRUMENT UND VERFAHREN ZUR BEFESTIGUNG EINER KLINGE**
BLADE FIXTURE FOR A SURGICAL INSTRUMENT AND METHOD FOR FASTENING A BLADE
MONTURE DE LAME POUR INSTRUMENT CHIRURGICAL ET PROCEDE DE FIXATION D'UNE LAME

(30) Priorität: 09.03.2001 DE 20104148 U
(43) Veröffentlichungstag der Anmeldung: 03.12.2003
(73) Patentinhaber: Schwind eye-tech-solutions GmbH & Co. KG, 63801 Kleinostheim (DE)
(72) Erfinder: KERN, Wolfgang, 63762 Grossostheim (DE)
(74) Vertreter: Hofstetter, Alfons J.
(86) Internationale Anmeldenummer: PCT/EP2002/000735
(87) Internationale Veröffentlichungsnummer: WO 2002/071990

(56) Entgegenhaltungen:
- EP-A- 0 432 325
- WO-A-01/91650
- WO-A-01/97729
- WO-A-98/48747
- US-A- 4 473 076

## Beschreibung

Die vorliegende Erfindung betrifft eine Klingenhalterung für ein chirurgisches Instrument, insbesondere für ein chirurgisches Schneideinstrument zum Schneiden und/oder zur Abtragung einer menschlichen oder tierischen Cornea, mit mindestens einer Klinge bestehend aus einer Schneide, zwei sich gegenüberliegenden Klingenrändern und einem der Schneide gegenüberliegenden Klingenende. Des weiteren betrifft die Erfindung ein Verfahren zur Befestigung einer Klinge bestehend aus einer Schneide, zwei sich gegenüberliegenden Klingenrändern und einem der Schneide gegenüberliegenden Klingenende in einer Klingenhalterung für ein chirurgisches Instrument, insbesondere für ein chirurgisches Schneideinstrument zum Schneiden und/oder zur Abtragung einer menschlichen oder tierischen Cornea. Schließlich betrifft die vorliegende Erfindung noch die Verwendung einer entsprechend neuen Klingenhalterung.

Derartige Klingenhalterungen sind bekannt. So werden in den internationalen Patentanmeldungen WO 98/48747, WO 00/56222 und WO 00/61015 gattungsgemäße Klingenhalterungen beschrieben. Dabei ist die Klinge trapezförmig, teilweise trapezförmig oder rechteckig ausgestaltet und weist mindestens eine in der Klinge ausgebildete Öffnung auf. Über einen entsprechenden Vorsprung der Klingenhalterung, der durch die genannte Öffnung hindurchgeschoben wird, kommt es zu einer Verbindung zwischen der Klingenhalterung und der Klinge. Nachteilig an dieser Befestigungsmethode ist jedoch, daß hierbei die Klinge nur unter Einwirkung von erheblichen Kräften mittels Quetschen, Stempeln, oder Ähnlichem auf die Klingenhalterung aufgebracht werden kann. Durch die erforderliche hohe Kraft- oder Hitzeeinwirkung kann hierbei die benötigte Präzision zwischen Klinge und Klingenhalterung nicht gewährleistet werden. Eine hohe Präzision ist insbesondere bei der Führung der Klinge innerhalb des Schneidkopfes alleinig durch den Klingenhalter unerlässlich. Zudem kann die Klinge oder die Klingenhalterung, insbesondere Vorsprünge der Klingenhalterung, beschädigt werden, so daß bei derartigen bekannten Klingenhalterungen bei der Produktion sehr hohe Ausschußquoten entstehen. Zudem erhöhen sich dadurch die Herstellungskosten einer einzelnen Klingenhalterung mit Klinge.

In diesem Zusammenhang sei noch auf die WO 01/91650 A verwiesen, deren Inhalt als Stand der Technik nach Artikel 54 (3) EPÜ gilt. Diese Druckschrift offenbart eine Klingenhalterung für eine Klinge, wobei die relative Position der Klingenhalterung abgeglichen werden kann, um den Abstand zwischen einer Bezugsfläche der Klingenhalterung und der Schneide der Klinge zu kontrollieren. Die Klinge besteht aus einer Schneide, zwei sich gegenüberliegenden Klingenrändern mit jeweils einer Einkerbung und einem der Schneide gegenüberliegenden Klingenende. Die Klingenhalterung hat eine ebene Oberfläche, an deren beiden einander gegenüberliegenden Seiten je eine Befestigungsklemme angeordnet ist. Zur Befestigung der Klinge wird diese mittels der Klemmen gegen die ebene Oberfläche der Klingenhalterung gedrückt.

Es ist Aufgabe der vorliegenden Erfindung, eine gattungsgemäße Klingenhalterung für ein chirurgisches Instrument bereitzustellen, welche einfach, präzise und sicher mit einer Klinge zu versehen ist und in Kombination mit der Klinge einfach herstellbar ist.

Es ist weiterhin Aufgabe der vorliegenden Erfindung ein gattungsgemäßes Verfahren zur Befestigung einer Klinge in einer Klingenhalterung bereitzustellen, welches ein einfaches, präzises und sicheres Befestigen der Klinge in der Klingenhalterung sowie die Auswechselbarkeit der Klinge gewährleistet.

Gelöst werden diese Aufgaben durch eine Klingenhalterung mit den Merkmalen des unabhängigen Anspruches 1 und ein Verfahren mit den Merkmalen des unabhängigen Anspruchs 14.

Vorteilhafte Ausgestaltungen sind in den Unteransprüchen beschrieben.

Eine erfindungsgemäße Klingenhalterung für ein chirurgisches Instrument weist zwei sich gegenüberliegende Führungen zur Aufnahme von Klingenrändern einer Klinge auf, wobei die Klinge durch ein Einklipsen in die Führungen mit der Klingenhalterung lösbar verbindbar ist und zwischen den Führungen der Klingenhalterung eine Auflagefläche zur Auflage der Klinge ausgebildet ist, wobei die Auflagefläche eine gekrümmte Oberfläche aufweist. Dadurch ist einerseits gewährleistet, daß die Klinge einfach mit der Klingenhalterung verbindbar ist und die Kombination der Klingenhalterung mit der Klinge somit auch einfach und kostengünstig herstellbar ist. Die Krafteinwirkung auf die Klinge wird durch das Einklipsen der Klinge minimiert. Es ist gewährleistet, daß die Klinge in keinem Fall den Klingenhalter verlassen kann, da hierzu die Klinge überbogen werden müßte, was innerhalb der Spalte bzw. der Klingenhalterung nicht möglich ist. Zudem ist eine hohe Präzision bei der Verbindung von Klinge und Klingenhalter gewährleistet.

In einer vorteilhaften Ausgestaltung der erfindungsgemäßen Klingenhalterung weisen die Führungen jeweils mindestens einen Spalt zur Aufnahme der Klingenränder der Klinge auf. Durch die Ausbildung von Spalten in den Führungen ist ein sicheres, präzises Einklipsen und Halten der Klinge in der Klingenhalterung gewährleistet.

In einer vorteilhaften Ausgestaltung der erfindungsgemäßen Klingenhalterung weist mindestens eine der Führungen mindestens einen Vorsprung auf, wobei der Vorsprung in eine entsprechende randliche Ausnehmung der Klinge eingreift. Dadurch ist einerseits ein sicherer Halt der Klinge in der Klingenhalterung wie auch eine hohe Präzision beim Zusammenfügen von Klinge und Klingenhalter gewährleistet.

In einer vorteilhaften Ausgestaltung der erfindungsgemäßen Klingenhalterung bestehen die Führungen aus jeweils mindestens einem im Bereich der Klingenhalterränder angeordneten Vorsprung, wobei die sich gegenüberliegenden Vorsprünge entsprechende Spalte zur Aufnahme der Klingenränder der Klinge aufweisen. Eine derartige Ausgestaltung minimiert den Platz- und Materialbedarf der Führungen, so daß der Klingenhalter insgesamt leicht und kleindimensioniert gehalten werden kann.

In einer vorteilhaften Ausgestaltung der erfindungsgemäßen Klingenhalterung sind die Führungen einstückig mit der Klingenhalterung ausgebildet. Es ist aber auch möglich, dass die Führungen aus separaten, mit der Klingenhalterung zu verbindenden Elementen bestehen. Dies bietet die Möglichkeit, dass die Führungen dreh- und oder verschiebbar ausgebildet sind und so an verschiedene Klingengrößen leicht anpassbar sind.

In einer weiteren vorteilhaften Ausgestaltung der Erfindung sind die den Führungen zugewandten Enden der gekrümmten oder ebenen Oberfläche der Auflagefläche von den Führungen beabstandet und enden überhalb einer Bodenfläche der jeweiligen Spalte. Dadurch erhöht sich die Klemmwirkung der Klinge innerhalb der Führungen des Klingenhalters deutlich. Zudem kann die Klinge biegsam ausgebildet sein, wodurch das Einklipsen der Klinge in die Führungen erleichtert wird.

In einer weiteren vorteilhaften Ausgestaltung der erfindungsgemäßen Klingenhalterung weisen die Klingenränder eine geringere Dicke auf als ein Klingenkörper der Klinge. Dadurch ist es möglich, den Vorgang des Einklipsen weiter zu vereinfachen und zu beschleunigen.

In einer weiteren vorteilhaften Ausgestaltung der erfindungsgemäßen Klingenhalterung ist die Klinge rechteckig ausgebildet und weist eine aus der Klingenhalterung hervortretende Schneide auf, wobei im Bereich der Schneide jeweils ein Abstandselement angeformt ist. Durch die Variation der Größe und Länge der Abstandselemente kann vorteilhafterweise vorbestimmt werden, um wie weit die Schneide aus der Klingenhalterung hervortritt.

Ein erfindungsgemäßes Verfahren zur Befestigung einer Klinge bestehend aus einer Schneide, zwei sich gegenüberliegenden Klingenrändern und einem der Schneide gegenüberliegenden Klingenende in einer Klingenhalterung für ein chirurgisches Instrument, insbesondere für ein chirurgisches Schneideinstrument zum Schneiden und/oder zur Abtragung einer menschlichen oder tierischen Cornea ist gekennzeichnet durch ein Einklipsen der Klingenränder der Klinge in zwei entsprechende Spalte von sich gegenüberliegenden Führungen der Klingenhalterung, wobei die Befestigung der Klinge an der Klingenhalterung lösbar ist und zwischen den Führungen der Klingenhalterung eine Auflagefläche zur Auflage der Klinge ausgebildet ist, wobei die Auflagefläche eine gekrümmte Oberfläche aufweist. Durch das erfindungsgemäße Verfahren ist ein einfaches und sicheres Befestigen der Klinge in der Klingenhalterung sowie die Auswechselbarkeit der Klinge gewährleistet.

Eine Verwendung einer erfindungsgemäßen Klingenhalterung betrifft das Befestigen einer Klinge in einem chirurgischen Schneideinstrument zum Schneiden und/oder zur Abtragung einer menschlichen oder tierischen Cornea.

Weitere Einzelheiten, Merkmale und Vorteile der Erfindung ergeben sich aus den in den folgenden Figuren dargestellten und beschriebenen Ausführungsbeispielen:

Es zeigen
- Figur 1: eine perspektivische Ansicht eines erfindungsgemäßen Klingenhalters mit eingelegter Klinge gemäß einer ersten Ausführungsform;
- Figur 2: eine perspektivische Ansicht des erfindungsgemäßen Klingenhalters gemäß Figur 1 ohne eingelegte Klinge;
- Figur 3: eine perspektivische Ansicht einer ersten Ausführungsform einer Klinge für einen erfindungsgemäßen Klingenhalter;
- Figur 4: eine perspektivische Ansicht eines erfindungsgemäßen Klingenhalters mit eingelegter Klinge gemäß einer zweiten Ausführungsform; und
- Figur 5: eine perspektivische Ansicht einer zweiten Ausführungsform einer Klinge für einen erfindungsgemäßen Klingenhalter.

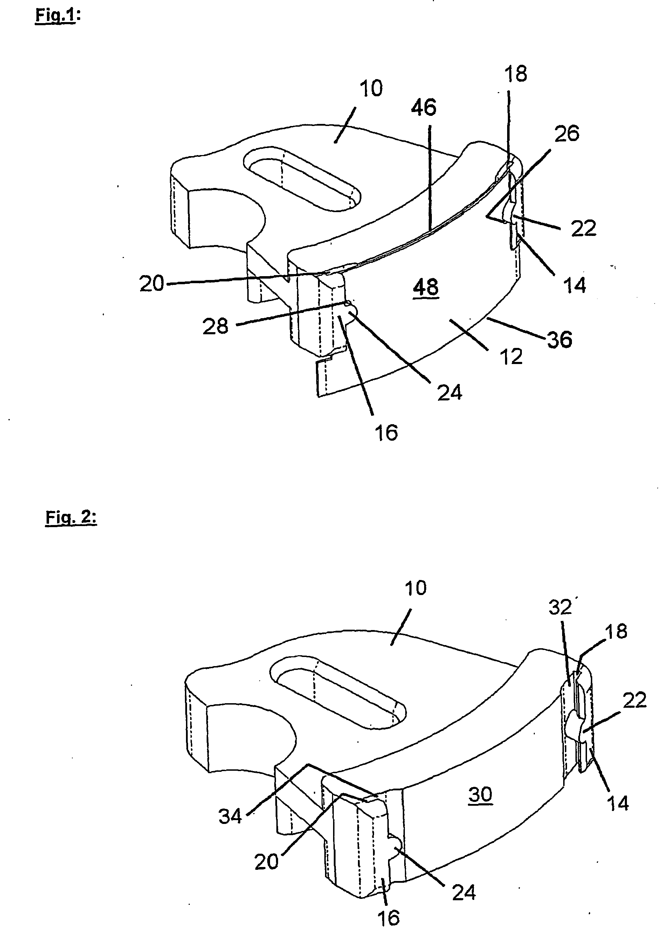

Figur 1 zeigt eine Klingenhalterung 10 für ein chirurgisches Instrument, insbesondere für ein chirurgisches Schneideinstrument zur Abtragung einer menschlichen oder tierischen Cornea, mit einer Klinge 12. Die Klinge 12 weist einen Klingenkörper 48 mit einer Schneide 36, zwei sich gegenüberliegenden Klingenrändern 42, 44 und einem der Schneide 36 gegenüberliegenden Klingenende 46 auf. Man erkennt, daß die Klingenhalterung 10 zwei sich gegenüberliegende Führungen 14, 16 mit entsprechenden Spalten 18, 20 zur Aufnahme der Klingenränder 42, 44, der Klinge 12 aufweist. Die Klinge 12 ist dabei biegsam ausgebildet und wird durch Einklipsen in die Spalte 18, 20 der Führungen 14, 16 mit der Klingenhalterung 10 lösbar verbunden. Die Klingenhalterung 10 kann dabei aus Metall, einer Metall-Legierung, aus Kunststoff oder Keramik bestehen. Die Klinge 12 besteht üblicherweise aus Metall, einer Metall-Legierung oder aus Keramik. Zudem kann sie biegsam ausgebildet sein.

Des weiteren erkennt man, daß die Führungen 14, 16 jeweils einen Vorsprung 22, 24 ausbilden. Die Vorsprünge 22, 24 greifen dabei jeweils in eine entsprechende randliche Ausnehmung 26, 28 der Klinge 12 ein.

Es ist auch möglich, dass gemäß einer nicht dargestellten Ausführungsform die Führungen 14, 16 aus jeweils mindestens einem im Bereich der Klingenhalterränder angeordneten Vorsprung bestehen, wobei die sich gegenüberliegenden Vorsprünge entsprechende Spalte zur Aufnahme der Klingenränder 42, 44 der Klinge 12 aufweisen.

Man erkennt, dass die Führungen 14, 16 einstückig mit der Klingenhalterung 10 ausgebildet sind. Es ist aber auch denkbar, dass gemäß einer weiteren Ausführungsform (nicht dargestellt) die Führungen 14, 16 aus separaten, mit der Klingenhalterung 10 zu verbindenden Elementen bestehen. In diesem Fall können die Führungen 14, 16 dreh- und oder verschiebbar ausgebildet sein.

Figur 2 zeigt eine Klingenhalterung 10 ohne eingelegter Klinge 12. Man erkennt, daß zwischen den Führungen 16 eine Auflagefläche 30 zur Auflage der Klinge 12 ausgebildet ist. Die Auflagefläche 30 weist dabei eine gekrümmte Oberfläche auf. Die den Führungen 14, 16 zugewandten Enden der gekrümmten Oberfläche der Auflagefläche 30 sind dabei von den Führungen 14, 16 beabstandet. Zudem enden sie überhalb einer Bodenfläche 32, 34 der jeweiligen Spalten 18, 20.

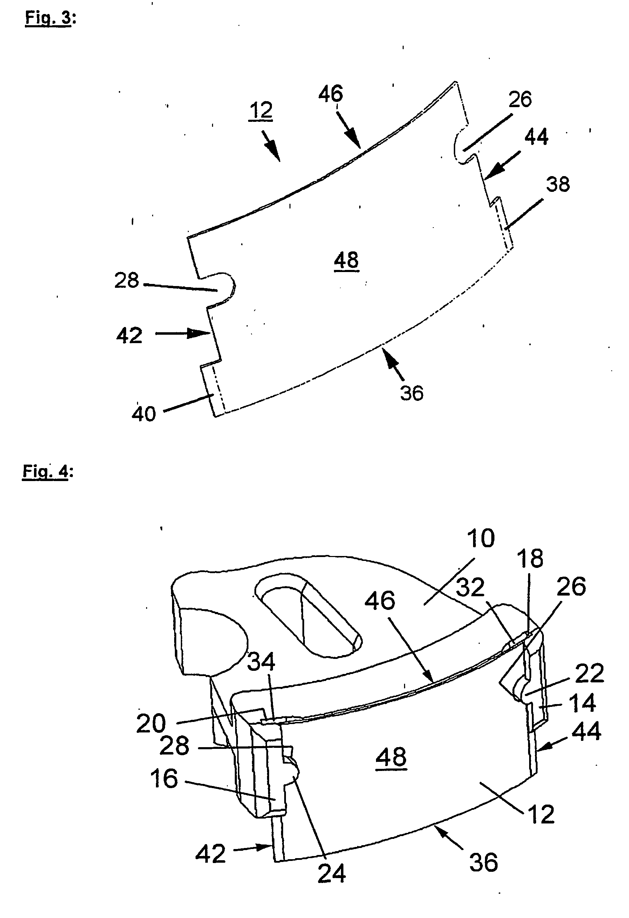

Figur 3 zeigt die Klinge 12 mit einer Schneide 36 und den beiden randlichen Ausnehmungen 26, 28 in den Klingenrändern 42, 44. Man erkennt, daß in dem dargestellten Ausführungsbeispiel die Klinge 12 grundsätzlich rechteckig und leicht gebogen ausgebildet ist, wobei im Bereich der Schneide 36 jeweils randlich ein Abstandselement 38, 40 angeformt ist. Die Größe der Abstandselemente 38, 40 bestimmen den Abstand der Schneide 36 zur Klingenhalterung 10. Die Klinge 12 kann aber auch jede andere geeignete Form wie z.B. trapezförmig, teilweise trapezförmig oder bogenförmig aufweisen. Zudem können auch nicht gebogene Klingen verwendet werden.

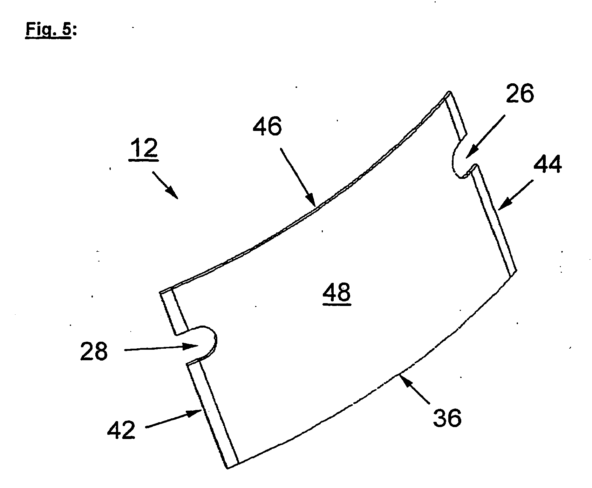

Figur 4 zeigt eine perspektivische Ansicht eines Klingenhalters 10 mit eingelegter Klinge 12 gemäß einer zweiten Ausführungsform. Man erkennt, dass im Vergleich zu der ersten Ausführungsform die Klinge 12 im Bereich der Schneide 36 keine Abstandselemente aufweist. Noch deutlicher wird diese Form der Klinge 12 in Figur 5. Es zeigt sich, dass die hier dargestellte Klinge 12 ein rechteckige Form aufweist. Zudem erkennt man, dass die Klingenränder 42, 44 eine geringere Dicke aufweisen als der Klingenkörper 48 der Klinge 12. Auch in diesem Ausführungsbeispiel der Klinge 12 weisen die Klingenränder 42, 44 jeweils eine entsprechende Ausnehmung 28, 26 zum Eingriff in korrespondierende Vorsprünge 24,22 auf.

## Patentansprüche

1. Klingenhalterung für ein chirurgisches Instrument, insbesondere für ein chirurgisches Schneideinstrument zum Schneiden und/oder zur Abtragung einer menschlichen oder tierischen Cornea, mit mindestens einer Klinge (12) bestehend aus einer Schneide (36), zwei sich gegenüberliegenden Klingenrändern (42, 44) und einem der Schneide (36) gegenüberliegenden Klingenende (46),
**dadurch gekennzeichnet,**
**daß** die Klingenhalterung (10) zwei sich gegenüberliegende Führungen (14, 16) zur Aufnahme der Klingenränder (42, 44) der Klinge (12) aufweist, wobei die Klinge (12) durch ein Einklipsen in die Führungen (14, 16) mit der Klingenhalterung (10) lösbar verbindbar ist, und
**daß** zwischen den Führungen (14, 16) der Klingenhalterung (10) eine Auflagefläche (30) zur Auflage der Klinge (12) ausgebildet ist, wobei die Auflagefläche (30) eine gekrümmte Oberfläche aufweist.

2. Klingenhalterung nach Anspruch 1,
**dadurch gekennzeichnet,**
**daß** die Führungen (14, 16) jeweils mindestens einen Spalt (18, 20) zur Aufnahme der Klingenränder (42, 44) der Klinge (12) aufweisen.

3. Klingenhalterung nach Anspruch 1 oder 2,
**dadurch gekennzeichnet,**
**daß** mindestens eine der Führungen (14, 16) mindestens einen Vorsprung (22, 24) aufweist, wobei der Vorsprung (22, 24) in eine entsprechende randliche Ausnehmung (26, 28) der Klingenränder (42, 44) der Klinge (12) eingreift.

4. Klingenhalterung nach Anspruch 1,
**dadurch gekennzeichnet,**
**daß** die Führungen (14, 16) aus jeweils mindestens einem im Bereich der Klingenhalterränder angeordneten Vorsprung bestehen, wobei die sich gegenüberliegenden Vorsprünge entsprechende Spalte zur Aufnahme der Klingenränder (42, 44) der Klinge (12) aufweisen.

5. Klingenhalterung nach einem der vorhergehenden Ansprüche,
**dadurch gekennzeichnet,**
**daß** die Führungen (14, 16) einstückig mit der Klingenhalterung (10) ausgebildet sind.

6. Klingenhalterung nach einem der Ansprüche 1 bis 4,
**dadurch gekennzeichnet,**
**daß** die Führungen (14, 16) aus separaten, mit der Klingenhalterung (10) zu verbindenden Elementen bestehen.

7. Klingenhalterung nach Anspruch 6,
**dadurch gekennzeichnet,**
**daß** die Führungen (14, 16) dreh- und oder verschiebbar ausgebildet sind.

8. Klingenhalterung nach einem der vorhergehenden Ansprüche,
**dadurch gekennzeichnet,**
**daß** die den Führungen (14, 16) zugewandten Enden der gekrümmten Oberfläche der Auflagefläche (30) von den Führungen (14, 16) beabstandet sind und überhalb einer Bodenfläche (32, 34) der jeweiligen Spalte (18, 20) enden.

9. Klingenhalterung nach einem der vorhergehenden Ansprüche,
**dadurch gekennzeichnet,**
**daß** die Klingenränder (42, 44) eine geringere Dicke aufweisen als ein Klingenkörper (48) der Klinge (12).

10. Klingenhalterung nach einem der vorhergehenden Ansprüche,
**dadurch gekennzeichnet,**
**daß** die Klinge (12) rechteckig ausgebildet ist und die Schneide (36) aus der Klingenhalterung (10) hervortritt, wobei im Bereich der Schneide (36) jeweils randlich ein Abstandselement (38, 40) angeformt ist.

11. Klingenhalterung nach einem der vorhergehenden Ansprüche,
**dadurch gekennzeichnet,**
**daß** die Klingenhalterung (10) aus Metall, einer Metall-Legierung, aus Keramik oder Kunststoff besteht.

12. Klingenhalterung nach einem der vorhergehenden Ansprüche,
**dadurch gekennzeichnet,**
**daß** die Klinge (12) aus Metall, einer Metall-Legierung oder aus Keramik besteht.

13. Klingenhalterung nach einem der vorhergehenden Ansprüche,
**dadurch gekennzeichnet,**
**daß** die Klinge (12) biegsam ausgebildet ist.

14. Verfahren zur Befestigung mindestens einer Klinge bestehend aus einer Schneide (36), zwei sich gegenüberliegenden Klingenrändern (42, 44) und einem der Schneide (36) gegenüberliegenden Klingenende (46) in einer Klingenhalterung (10) für ein chirurgisches Instrument, insbesondere für ein chirurgisches Schneideinstrument zum Schneiden und/oder zur Abtragung einer menschlichen oder tierischen Cornea,
**dadurch gekennzeichnet,**
**dass** durch ein Einklipsen der Klingenränder (42, 44) der Klinge (12) in zwei entsprechende Spalte (18, 20) von sich gegenüberliegenden Führungen (14, 16) der Klingenhalterung (10) die Klinge (12) lösbar an der Klingenhalterung (10) befestigt wird, wobei zwischen den Führungen (14, 16) der Klingenhalterung (10) eine Auflagefläche (30) mit gekrümmter Oberfläche zur Auflage der Klinge (12) ausgebildet ist.

15. Verwendung einer Klingenhalterung nach den Ansprüchen 1 bis 11 zum Befestigen einer Klinge in einem chirurgischen Schneideinstrument zum Schneiden und/oder zur Abtragung einer menschlichen oder tierischen Cornea.

## Claims

1. Blade holder for a surgical instrument, especially for a surgical cutting instrument for cutting and/or ablating a human or animal cornea, including at least one blade (12) consisting of an edge (36), two blade peripheries (42, 44) opposing each other, and a blade end (46) opposing the edge (36),
**characterized in that**
the blade holder (10) has two guides (14, 16) opposing each other for receiving the blade peripheries (42, 44) of the blade (12), wherein the blade (12) is releasably connectable to the blade holder (10) by clipping into the guides (14, 16), and **in that** a supporting surface (30) for supporting the blade (12) is formed between the guides (14, 16) of the blade holder (10), wherein the supporting surface (30) has a curved surface.

2. Blade holder according to claim 1,
**characterized in that**
the guides (14, 16) each have at least one gap (18, 20) for receiving the blade peripheries (42, 44) of the blade (12).

3. Blade holder according to claim 1 or 2,
**characterized in that**
at least one of the guides (14, 16) has at least one projection (22, 24), wherein the projection (22, 24) engages a corresponding peripheral recess (26, 28) of the blade peripheries (42, 44) of the blade (12).

4. Blade holder according to claim 1,
**characterized in that**
the guides (14, 16) are each composed of at least one projection disposed in the region of the blade holder peripheries, wherein the projections opposing each other have corresponding gaps for receiving the blade peripheries (42, 44) of the blade (12).

5. Blade holder according to anyone of the preceding claims,
**characterized in that**
the guides (14, 16) are formed integrally with the blade holder (10).

6. Blade holder according to anyone of claims 1 to 4,
**characterized in that**
the guides (14, 16) are composed of separate elements to be connected to the blade holder (10).

7. Blade holder according to claim 6,
**characterized in that**
the guides (14, 16) are formed in rotatable and/or displaceable manner.

8. Blade holder according to anyone of the preceding claims,
**characterized in that**
the ends of the curved surface of the supporting surface (30) facing the guides (14, 16) are spaced apart from the guides (14, 16) and terminate above a bottom surface (32, 34) of the respective gaps (18, 20).

9. Blade holder according to anyone of the preceding claims,
**characterized in that**
the blade peripheries (42, 44) have a lower thickness than a blade body (48) of the blade (12).

10. Blade holder according to anyone of the preceding claims,
**characterized in that**
the blade (12) is rectangularly formed and the edge (36) protrudes from the blade holder (10), wherein a spacer (38, 40) is respectively formed peripherally in the region of the edge (36).

11. Blade holder according to anyone of the preceding claims,
**characterized in that**
the blade holder (10) is made of metal, a metal alloy, of ceramics or plastics.

12. Blade holder according to anyone of the preceding claims,
**characterized in that**
the blade (12) is made of metal, a metal alloy or of ceramics.

13. Blade holder according to anyone of the preceding claims,
**characterized in that**
the blade (12) is flexibly formed.

14. Method for attaching at least one blade consisting of an edge (36), two blade peripheries (42, 44) opposing each other and a blade end (46) opposing the edge (36), in a blade holder (10) for a surgical instrument, especially for a surgical cutting instrument for cutting and/or ablating a human or animal cornea,
**characterized in that**
by clipping the blade peripheries (42, 44) of the blade (12) into two corresponding gaps (18, 20) of guides (14, 16) of the blade holder (10) opposing each other, the blade (12) is releasably attached to the blade holder (10), wherein a supporting surface (30) with a curved surface for supporting the blade (12) is formed between the guides (14, 16) of the blade holder (10).

15. Use of a blade holder according to claims 1 to 11 for attaching a blade in a surgical cutting instrument for cutting and/or ablating a human or animal cornea.

## Revendications

1. Monture de lame pour instrument chirurgical, en particulier pour instrument chirurgical de coupe pour couper et/ou pour l'ablation d'une cornée humaine ou animale, comportant au moins une lame (12) se composant d'un fil (36), de deux bords de lame se faisant face à face (42, 44) et d'une extrémité de lame (46) en face du fil (36)
**caractérisée en ce que**
la monture de lame (10) présente deux guides (14, 16) se faisant face à face pour recevoir les bords de lame (42, 44) de la lame (12), la lame (12) pouvant se solidariser de façon amovible avec la monture de lame (10) par clipsage dans les guides (14, 16) et qu'entre les guides (14, 16) de la monture de lame (10) une surface support (30) pour supporter la lame (12) est formée, la surface support (30) présentant une surface supérieure courbée.

2. Monture de lame selon la revendication 1,
**caractérisée en ce que**
les guides (14, 16) présentent chaque fois au moins une fente (18, 20) pour recevoir les bords de lame (42, 44) de la lame (12).

3. Monture de lame selon la revendication 1 ou 2,
**caractérisée en ce que**
au moins un des guides (14, 16) présente au moins une saillie (22, 24), la saillie (22, 24) s'engageant dans un creux correspondant de bord (26, 28) des bords de lame (42, 44) de la lame (12).

4. Monture de lame selon la revendication 1,
**caractérisée en ce que**
les guides (14, 16) se constituent chaque fois d'au moins une saillie figurant dans la zone des bords de maintien de lame, les saillies se faisant face à face présentant des fentes correspondantes pour recevoir les bords de lame (42, 44) de la lame (12).

5. Monture de lame selon l'une quelconque des revendications précédentes,
**caractérisée en ce que**
les guides (14, 16) sont monoblocs avec la monture de lame (10).

6. Monture de lame selon l'une quelconque des revendications 1 à 4,
**caractérisée en ce que**
les guides (14, 16) se constituent d'éléments séparés, à solidariser avec la monture de lame (10).

7. Monture de lame selon la revendication 6,
**caractérisée en ce que**
les guides (14, 16) sont réalisés de façon à pouvoir pivoter et/ou coulisser.

8. Monture de lame selon l'une quelconque des revendications précédentes,
**caractérisée en ce que**
les extrémités de la surface courbée dirigées vers les guides (14, 16) de la surface support (30) sont distantes des guides (14, 16) et aboutissent au-dessus d'une surface de fond (32, 34) des fentes (18, 20) correspondantes.

9. Monture de lame selon l'une quelconque des revendications précédentes,
**caractérisée en ce que**
les bords de lame (42, 44) sont d'une épaisseur inférieure à celle d'un corps de lame (48) de la lame (12).

10. Monture de lame selon l'une quelconque des revendications précédentes,
**caractérisée en ce que**
la lame (12) est de forme rectangulaire et que le fil (36) dépasse de la monture de lame (10), un élément d'écartement (38, 40) étant chaque fois formé au bord, dans la zone du fil (36).

11. Monture de lame selon l'une quelconque des revendications précédentes,
**caractérisée en ce que**
la monture de lame (10) est en métal, en un alliage métallique, en céramique ou en matière synthétique.

12. Monture de lame selon l'une quelconque des revendications précédentes,
**caractérisée en ce que**
la lame (12) est en métal, en un alliage métallique ou en céramique.

13. Monture de lame selon l'une quelconque des revendications précédentes,
**caractérisée en ce que**
la lame (12) est réalisée de façon à être flexible.

14. Procédé de fixation d'au moins une lame se constituant d'un fil (36), de deux bords de lame se faisant face à face (42, 44) et d'une extrémité de lame (46) en face du fil (36) dans une monture de lame (10) pour instrument chirurgical, en particulier pour instrument chirurgical de coupe pour couper et/ou pour l'ablation d'une cornée humaine ou animale,
**caractérisé en ce que**
par clipsage des bords de lame (42, 44) de la lame (12) dans deux fentes correspondantes (18, 20) de guides (14, 16) se faisant face à face de la monture de lame (10), la lame (12) est solidarisée de façon amovible avec la monture de lame (10), une surface support (30) présentant une surface supérieure courbée pour supporter la lame (12) étant formée entre les guides (14, 16) de la monture de lame (10).

15. Utilisation d'une monture de lame selon les revendications 1 à 11 pour solidariser une lame à un instrument chirurgical pour couper et/ou réaliser l'ablation d'une cornée humaine ou animale.
